# FASCICULE DE BREVET EUROPEEN

(11) **EP 0 668 257 B1**
(45) Date de publication et mention de la délivrance du brevet: **15.04.1998**
(21) Numéro de dépôt: 95400265.5
(22) Date de dépôt: 07.02.1995
(51) Int. Cl.: C07C 13/18, C07C 5/10, B01J 23/78

(54) **Catalyseur et procédé d'hydrogénation du benzène utilisant ledit catalyseur**
Katalysator sowie Verfahren zur Hydrierung von Benzol in dem dieser Katalysator angewendet wird
Catalyst and process for the hydrogenation of benzene using the said catalyst

(30) Priorité: 17.02.1994 FR 9401916
(43) Date de publication de la demande: 23.08.1995
(73) Titulaire: INSTITUT FRANCAIS DU PETROLE, 92502 Rueil-Malmaison (FR)
(72) Inventeur: Forestiere, Alain, F-69390 Vernaison (FR); Delhomme, Henri, F-69110 Sainte Foy Les Lyons (FR); Yout, Pierre, F-38200 Vienne (FR)

(56) Documents cités:
- US-A- 3 840 608
- US-A- 4 357 478
- CHEMICAL ABSTRACTS, vol. 113, no. 23, 3 Décembre 1990 Columbus, Ohio, US; abstract no. 211547y, page 722; & CN-A-1 037 729

## Description

La présente invention concerne un catalyseur homogène à base de nickel utilisable en particulier pour l'hydrogénation du benzène.

La présente invention concerne également un procédé de production du cyclohexane, par hydrogénation du benzène, se déroulant en au moins deux étapes. Au cours de la première étape l'hydrogénation du benzène est effectuée en phase liquide en présence d'une suspension colloïdale d'un catalyseur à base de nickel et dans des conditions de pression et de températures choisies de manière à ce que l'on récupère une phase vapeur contenant au moins une partie du cyclohexane formé. Au cours de la deuxième étape la phase vapeur obtenue à la première étape est envoyée dans une zone dite zone d'hydrogénation de finition au cours de laquelle les produits hydrogénables contenus dans cette phase vapeur sont hydrogénés en présence d'un catalyseur solide.

La réaction d'hydrogénation du benzène en cyclohexane est une réaction fortement exothermique et est donc favorisée par une basse température et une forte pression partielle d'hydrogène. De nombreux procédés industriels ont été développés et peuvent se différencier par les conditions opératoires, suivant que la réaction a lieu en phase liquide ou en phase gazeuse. Ces procédés se différencient également par la nature du catalyseur et par la méthode utilisée pour compenser l'élévation de température, due à l'exothermicité de la réaction.

Parmi les procédés les plus connus, opérant en phase liquide, on peut citer le procédé Hydrar développé par la société Union Oil Product qui utilise un lit fixe de catalyseur à base de platine. Dans ce procédé le benzène de charge et un recyclage de cyclohexane, mélangés à l'hydrogène frais ou recyclé après recompression à la pression nécessaire, sont préchauffés, puis introduits dans une série de deux ou trois réacteurs, dont les températures s'étagent entre 200 °C et 300 °C et qui opère sous 3 mégapascals (MPa) environ. La conversion par passe du benzène est pratiquement complète. L'effluent des réacteurs est refroidi par échange thermique avec la charge et une partie du liquide est renvoyée comme diluant afin de faciliter le contrôle de la température. On peut aussi citer le procédé Houdry utilisant trois réacteurs en série opérant en lits fixes avec des recyclages de cyclohexane ; le procédé Sinclair-Engelhard utilisant un seul réacteur dans lequel l'élimination de la chaleur se fait in situ à l'aide d'un faisceau tubulaire avec production de vapeur et dans lequel il n'y a pas de recyclage de cyclohexane. On peut encore citer le procédé B.P. qui effectue l'hydrogénation en deux étapes successives. Dans ce procédé l'effluent de la première étape contient environ 95 % en poids de cyclohexane et le contrôle de la température de réaction est assuré par des recyclages de liquide et de vapeur. Dans ce procédé le contrôle de la température utilisant à la fois la chaleur sensible et celle de vaporisation du cyclohexane recyclé, le taux de recirculation peut être largement abaissé, mais par contre la pression partielle d'hydrogène se trouve diminuée ce qui implique l'utilisation d'un deuxième réacteur dit réacteur de finition pour compléter l'hydrogénation. Enfin on peut citer le procédé de l'Institut Français du Pétrole (IFP) dans lequel la réaction s'effectue en phase liquide à une température d'environ 185 °C sous une pression de 2 à 3,5 MPa en présence d'un catalyseur à base de nickel maintenu en suspension par agitation à l'aide d'une circulation externe. Le produit hydrogéné quitte le réacteur en phase vapeur, ce qui contribue ainsi à faciliter l'évacuation d'une partie des calories. L'autre partie de la chaleur dégagée par la réaction est récupérée dans un échangeur disposé sur le circuit extérieur (circulation de la phase liquide servant à l'agitation) et utilisée à la production de vapeur basse pression. Le principal inconvénient de ce procédé provient du fait que le catalyseur utilisé est un nickel plus ou moins pyrophorique, dont les particules colloïdales sont relativement grosses ce qui limite à la fois son activité et la stabilité de la suspension.

Pour pouvoir utiliser ou vendre du cyclohexane il est aujourd'hui impératif d'obtenir un produit très pur contenant moins de 500 ppm d'impureté totale et en particulier de respecter une norme en vigueur dans la majorité des pays industrialisés concernant la teneur en benzène qui doit être inférieure à environ 100 ppm. Le respect de ces critères impose de choisir une température relativement basse (par exemple inférieure à environ 300 °C) et un catalyseur ne favorisant pas l'isomérisation du cyclohexane en méthyl-cyclopentane ni les réactions d'hydrocraquages. Lorsque l'on utilise un catalyseur à base de nickel l'isomérisation n'apparaît qu'à partir de 250 °C.

On a découvert de façon inattendue un procédé de production du cyclohexane par hydrogénation du benzène permettant de respecter les spécifications requises pour le cyclohexane produit. Ce procédé utilise dans sa première étape un catalyseur à base de nickel plus actif et plus stable que celui utilisé dans le procédé décrit dans le brevet US-A-4 357 478.

La présente invention concerne ainsi un procédé de production du cyclohexane, par hydrogénation du benzène, comprenant au moins les deux étapes, de préférence successives, suivantes :
a) on introduit progressivement la charge de benzène à hydrogéner et un gaz riche en hydrogène dans une zone réactionnelle contenant une phase liquide riche en cyclohexane et un catalyseur à base de nickel en suspension dans cette phase, ladite zone réactionnelle étant maintenue à une température d'environ 100 °C à environ 250 °C, sous une pression absolue d'environ 0,5 MPa à environ 10 MPa et on récupère une phase gazeuse contenant du cyclohexane, de rhydrogène et du benzène,
b) on introduit la phase gazeuse obtenue à l'étape a) dans un réacteur contenant au moins un lit fixe d'un catalyseur solide d'hydrogénation à base de nickel, ledit réacteur étant maintenu à une température de 100 °C à 300 °C, sous une pression absolue de 0,5 MPa à 10 MPa, la vitesse volumique horaire étant comprise entre 1 h⁻¹ et 10 h⁻¹ et on récupère après détente et refroidissement le cyclohexane sensiblement pur formé,
   ledit procédé étant caractérisé en ce que le catalyseur à base de nickel employé à l'étape a) est un produit qui résulte de la réduction par au moins un trialkylaluminium de formule AIR¹R²R³, dans laquelle R¹, R² et R³ représentent chacun indépendamment les uns des autres un groupe alkyle linéaire ou ramifié ayant de 1 à 12 atomes de carbone et le plus souvent de 1 à 4 atomes de carbone, d'une solution dans un hydrocarbure ou un mélange d'hydrocarbures d'au moins un carboxylate de nickel de formule R⁴COONi, dans laquelle R⁴ est un reste hydrocarboné ayant de 1 à 24 atomes de carbone et le plus souvent de 6 à 12 atomes de carbone, et d'au moins un carboxylate de sodium de formule R⁵COONa laquelle R⁵ est un reste hydrocarboné ayant de 1 à 24 atomes de carbone et le plus souvent de 6 à 12 atomes de carbone, le rapport molaire Ni : Na étant compris entre 2 : 1 et 1000 : 1 et de préférence entre 2 : 1 et 500 :1. R⁴ et R⁵ sont habituellement des restes hydrocarbonés aliphatiques et de préférence des groupes alkyles linéaires ou ramifiés.

Les sels de nickel et de sodium sont habituellement utilisés en solution dans un hydrocarbure ou un mélange d'hydrocarbures de point d'ébullition initial supérieur à 100 °C, de préférence supérieur à 140 °C et le plus souvent d'au moins 160 °C . On préfère généralement employer un mélange formé d'une coupe d'hydrocarbures saturés et de cyclohexane.

La solution utilisée à l'étape a) comprend le plus souvent au moins 30 % en poids de cylohexane et de préférence au moins 40 % en poids de cyclohexane et le plus souvent au moins 50 % en poids de cyclohexane par rapport au poids total du cyclohexane et de la coupe d'hydrocarbures utilisée.

La présente invention concerne également un catalyseur à base de nickel, notamment pour la mise en oeuvre du procédé de préparation du cyclohexane, formé par une suspension, dans un hydrocarbure ou un mélange d'hydrocarbures, résultant de la réduction par au moins un trialkylaluminium de formule AIR¹R²R³, dans laquelle R¹, R² et R³ ont la définition donnée ci-devant, d'une solution dans un hydrocarbure ou un mélange d'hydrocarbures d'au moins un carboxylate de nickel de formule R⁴COONi dans laquelle R⁴ a la définition donnée ci-devant, et d'au moins un carboxylate de sodium de formule R⁵COONa laquelle R⁵ a la définition donnée ci-devant, le rapport molaire Ni : Na étant compris entre 2 : 1 et 1000 : 1 et de préférence entre 2 : 1 et 500 :1. On utilise habituellement une suspension dans un mélange d'hydrocarbures comprenant de préférence du cyclohexane dans les proportions indiquées ci-devant pour la solution de l'étape a) du procédé.

Le plus souvent le trialkylaluminium employé est soit du triéthylaluminium soit du triisobutylaluminium. Les carboxylates de nickel et de sodium employés sont habituellement des carboxylates dérivés d'acides carboxyliques aliphatiques. On emploie le plus souvent un carboxylate de nickel et un carboxylate de sodium dérivant du même acide carboxylique. Le composé de nickel employé est généralement un octoate de nickel tel que par exemple l'éthyl-2 hexanoate de nickel et le composé de sodium est lui aussi un octanoate et par exemple l'éthyl-2 hexanoate de sodium. La réduction de ces sels est habituellement effectuée sous pression sensiblement atmosphérique, à une température inférieure ou égale à la température d'ébullition du cyclohexane. On utilise habituellement un excès de trialkylaluminium. Le rapport molaire de l'aluminium sur la somme nickel plus sodium est le plus souvent de 1,1 : 1 à 10 : 1 et de préférence de 1,5 :1 à 4 : 1. Le produit de cette première étape peut être rendu plus actif dans une étape subséquente qui consiste à chauffer la suspension à une température supérieure à environ 100 °C sous une pression supérieure à la pression atmosphérique et par exemple à environ 175 °C sous une pression de 0,8 Mpa. Ce chauffage à une durée suffisante pour que l'activité du catalyseur soit améliorée à titre d'exemple le chauffage pourra avoir une durée d'au moins une heure et par exemple de deux heures. Après refroidissement le catalyseur à base de nickel réduit se présente sous la forme d'une suspension colloïdale, de couleur noire, dans le cyclohexane. Cette suspension colloïdale, non abrasive, qui peut être manipulée par pompage, est habituellement utilisée telle quelle dans la première étape du procédé selon l'invention. Les quantités de sels de nickel et de sels de sodium utilisées sont habituellement choisies de manière à ce que la teneur finale en poids de nickel de la solution dans le cyclohexane soit de 1 % à 10 % et le plus souvent de 2 % à 8 % et la teneur en poids de sodium de cette solution de 0,02 % à 5 % et le plus souvent de 0,04 % à 2 %.

Un réacteur de finition traite l'effluent gazeux non transformé et peut assurer la régularité et la qualité de la production. Le rendement est pratiquement stoechiométrique et la pureté du produit obtenu est principalement liée à celle du benzène de départ.

Les produits gazeux issus de la première étape sont habituellement envoyés directement dans une deuxième étape, dans laquelle une hydrogénation, dite hydrogénation de finition, est réalisée dans un réacteur contenant au moins un lit fixe d'un catalyseur solide d'hydrogénation à base de nickel. Cette hydrogénation est réalisée en phase vapeur en présence du catalyseur de préférence déposé sur un support solide tel que par exemple un support à base d'alumine ou de silice. Ce catalyseur solide contient du nickel de préférence sous forme oxydée. L'utilisation d'un catalyseur contenant du nickel sous forme oxydée permet de réduire largement le risque de formation de pentane, d'hexane ou de méthylcyclopentane à partir du benzène encore présent dans la phase vapeur quittant l'étape a). De façon préférée le catalyseur comprend sous forme d'oxyde au moins 60 % en poids et le plus souvent de 75 à 95 % en poids du poids total de nickel présent. L'oxyde de nickel peut être présent à raison de 1 à 50 % en poids par rapport à la masse totale du catalyseur. Le dosage de la phase oxydée et de la phase métallique peut être effectuée par diffraction des rayons X ou par chimisorption d'hydrogène sur la phase métallique présente.

Le catalyseur solide de l'étape b) peut être préparé par toutes méthodes classiques bien connues de l'homme du métier. Ce catalyseur peut ainsi être préparé par imprégnation, malaxage, coprécipitation des sels précurseurs utilisés avec le support utilisé. Ce catalyseur pourra être prétraité par l'hydrogène à basse température, par exemple entre 150 °C et 300 °C, de manière à obtenir la forme catalytique désirée et en particulier le taux d'oxyde souhaité.

### Exemple 1 (comparatif)

On mélange 10 litres (7,79 kg) de cyclohexane pur et 7,34 kg d'une solution S₁ d'octoate de nickel à 13 % en poids de nickel. La solution S₁ est une solution d'octoate de nickel dans une coupe d'hydrocarbures saturés, de point d'ébullition initial supérieur à 160 °C, contenant moins de 1 % en poids d'eau. Après mélange on obtient 15,13 kg d'une solution S₂ contenant 0,954 kg (16,3 moles) de nickel.

Dans un réacteur en acier inoxydable, équipé d'un système d'agitation efficace et d'un système de refroidissement performant, on introduit 6,21 kg (54,5 moles) de triéthylaluminium, puis sous pression atmosphérique, et en contrôlant la température et le débit de gaz effluent, la solution S₂.

La vitesse d'addition de la solution S₂ est régulée de manière à ce que la température du milieu réactionnel reste égale à la température d'ébullition du cyclohexane dans les conditions de pression choisies. Le rapport molaire Al/Ni est de 3,4. La durée de l'addition est de deux heures. L'addition terminée, le mélange résultant est porté à 180 °C pendant encore 2 heures sous une pression contrôlée de 0,8 Mpa de manière à procéder à l'activation du catalyseur.

Après refroidissement et retour à la pression atmosphérique, on recueille 15,9 kg d'une suspension colloïdale de catalyseur dont la teneur en nickel est de 6 % en poids et la teneur en aluminium de 9,25 % en poids.

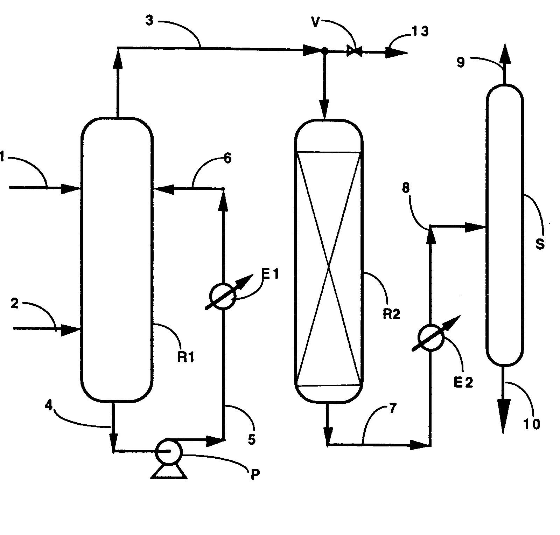

Une partie de cette suspension catalytique est utilisée pour hydrogéner du benzène en continu dans une petite unité pilote telle que schématisée sur la figure ci-après.

La suspension catalytique, 100 g (soit 6 g ou 0.1 mole de nickel) est introduite dans le réacteur R1 dans lequel on a préalablement placé du cyclohexane jusqu'à un niveau prédéterminé (80 % en volume du réacteur). Le contenu du réacteur est alors préchauffé sous agitation (par circulation de liquide à l'aide de la pompe P) vers 180 °C sous une pression d'hydrogène de3 Mpa.

On commence alors l'introduction par la ligne 1 de benzène (dont la teneur en soufre est de 0,2 ppm poids et la teneur en eau de 50 ppm poids) avec un débit de 108 grammes par heure (g/h) soit un débit molaire de 1,38 mole par heure.

En même temps et par la ligne 2, on introduit un gaz contenant 95% en volume d'hydrogène, 4,80% en volume d'hydrocarbures de C1 à C5 et 0,2% en volume d'azote et ceci à raison de 105 litres par heure (l/h) ce qui correspond à un débit molaire d'environ 4,2 mole/h d'hydrogène. Le rapport molaire hydrogène/benzène est alors voisin de 3,04 : 1.

Le liquide récupéré au fond du réacteur R1 par la ligne 4 est pompé à l'aide de la pompe P puis envoyé par la ligne 5 dans un échangeur de chaleur E1 et renvoyé dans le réacteur R1 par la ligne 6. Au cours de l'hydrogénation on maintient dans le réacteur R1 la température à environ 180 °C et la pression à 3 MPa. La phase vapeur contenant de l'hydrogène, un peu de benzène non converti et du cyclohexane est envoyé par la ligne 3 dans le réacteur de finition R2 qui contient un catalyseur solide à base de nickel. Ce catalyseur a été préparé par imprégnation à l'aide de nitrate de nickel de billes d'alumine dont la surface spécifique est de 250 m²/g et le volume poreux total de 0,65 cm³/g suivie d'un séchage et d'une calcination à 300 °C pendant 6 heures. La teneur en nickel du catalyseur est de 15 % en poids. Avant son utilisation dans le réacteur R2 pour l'hydrogénation de la phase gazeuse provenant du réacteur R1 ce catalyseur est traité par de l'hydrogène à 250 °C pendant 15 heures à une pression de 0,2 MPa et avec un débit de 800 litres/litre de catalyseur. Lors de l'hydrogénation de la phase gazeuse provenant du réacteur R1 par la ligne 3 le réacteur R2 travaille dans les conditions suivantes :
- Pression partielle d'hydrogène :: 0,6 MPa
- VVH (vitesse volumique horaire): 2
- Température d'entrée: 180 °C
- Température maximum dans le réacteur: 240 °C

L'effluent du réacteur R2 est envoyé par la ligne 7 dans un échangeur de chaleur E2 où il est refroidi, puis par la ligne 8 dans une colonne de séparation haute pression dans laquelle on sépare une phase gazeuse que l'on récupère par la ligne 9 et une phase liquide formée de cyclohexane très pur que l'on récupère par la ligne 10. Le benzène employé au cours de ce test est un benzène de haute pureté caractérisé par un point de cristallisation de 5, 5 °C correspondant à une teneur totale en impuretés de l'ordre de 400 ppm dont 280 ppm d'hydrocarbures saturés en C6 et C7, 100 ppm de cyclohexane et 20 ppm de toluène. On prélève par intermittence par la ligne 13 en ouvrant la vanne V (pour analyse) une petite quantité de la phase vapeur sortant du réacteur R1 par la ligne 3. Cette fraction est refroidie et on sépare une phase liquide contenant du cyclohexane et du benzène, d'une phase gazeuse contenant essentiellement de l'hydrogène. Durant les 15 premiers jours de fonctionnement la pureté du cyclohexane est excellente et reste supérieure à 99 %. Au bout de 2 mois de fonctionnement le cyclohexane sortant du réacteur R1 par la ligne 3 contient environ 1000 ppm de benzène. La teneur en benzène du cyclohexane récupéré par la ligne 10 reste en permanence en dessous de 30 ppm pendant toute la durée du test. Le cyclohexane récupéré par la ligne 10 au bout des 2 mois de test a un point de cristallisation de 6,40 °C. Le cyclohexane récupéré contient 5 ppm en poids d'hydrocarbures saturés en C5, 300 ppm d'hydrocarbures saturés en C6 et C7 dont 20 ppm de méthylcyclopentane.

### Exemple 2

On répète l'exemple 1 en utilisant dans le réacteur R1 un catalyseur contenant 600 ppm de sodium obtenu comme suit.

On mélange 10 litres (7,79 kg) de cyclohexane pur, 7,34 kg d'une solution S₁ d'octoate de nickel à 13 % en poids de nickel et 80 grammes d'une solution S₃ à 12 % en poids de sodium. La solution S₁ est une solution d'octoate de nickel dans une coupe d'hydrocarbures saturés, de point d'ébullition initial supérieur à 160 °C, contenant moins de 1 % en poids d'eau. La solution S₃ est une solution d'octoate de sodium dans une coupe d'hydrocarbures saturés, de point d'ébullition initial supérieur à 160 °C, contenant moins de 1 % en poids d'eau. Après mélange on obtient 15,21 kg d'une solution S₄ contenant 0,954 kg (16,3 moles) de nickel et 9,6 grammes (0,42 mole) de sodium.

Dans un réacteur en acier inoxydable, équipé d'un système d'agitation efficace et d'un système de refroidissement performant, on introduit 6,21 kg (54,5 moles) de triéthylaluminium, puis sous pression atmosphérique et en contrôlant la température et le débit de gaz effluent la solution S₄.

La vitesse d'addition de la solution S₄ est régulée de manière à ce que la température du milieu réactionnel reste égale à la température d'ébullition du cyclohexane dans les conditions de pression choisies. Le rapport molaire Al/Ni est de 3,4. Le rapport molaire Ni/Na est de 38,7. La durée de l'addition est de deux heures. L'addition terminée, le mélange résultant est porté à 180 °C pendant 2 heures sous une pression contrôlée de 0,8 Mpa de manière à procéder à l'activation du catalyseur.

Après refroidissement et retour à la pression atmosphérique, on recueille 16 kg d'une suspension colloïdale de catalyseur dont la teneur en nickel est de 6 % en poids, la teneur en aluminium est de 9,25 % en poids et la teneur en sodium de 600 ppm en poids.

Une partie de cette suspension catalytique est utilisée pour hydrogéner du benzène en continu dans une petite unité pilote telle que schématisée sur la figure ci-après.

La suspension catalytique, 100 g (soit 6 g ou 0,1 mole de nickel et 0,06 gramme ou 0,0023 mole de sodium) est introduite dans le réacteur R1 dans lequel on a préalablement placé du cyclohexane jusqu'à un niveau prédéterminé (80 % en volume du réacteur). Le contenu du réacteur est alors préchauffé sous agitation (par circulation de liquide à l'aide de la pompe P) vers 180 °C sous une pression d'hydrogène de3 Mpa.

On commence alors l'introduction par la ligne 1 de benzène (dont la teneur en soufre est de 0,2 ppm poids et la teneur en eau de 50 ppm poids) avec un débit de 108 grammes par heure (g/h) soit un débit molaire de 1,38 mole par heure. On opère la réduction dans les mêmes conditions que dans l'exemple 1. Au bout de 2 mois de fonctionnement le cyclohexane sortant du réacteur R1 par la ligne 3 contient moins de 200 ppm de benzène. La teneur en benzène du cyclohexane récupéré par la ligne 10 reste en permanence en dessous de 10 ppm pendant toute la durée du test. Le cyclohexane récupéré par la ligne 10 au bout des 2 mois de test a un point de cristallisation de 6,46 °C. Le cyclohexane récupéré contient, 290 ppm d'hydrocarbures saturés en C6 et C7 dont 10 ppm de méthylcyclopentane.

### Exemple 3

On répète l'exemple 1 en utilisant dans le réacteur R1 un catalyseur contenant 1 % en poids de sodium obtenu comme suit.

On mélange 10 litres (7,79 kg) de cyclohexane pur, 7,34 kg d'une solution S₁ d'octoate de nickel à 13 % en poids de nickel et 1,37 kg d'une solution S₃ à 12 % en poids de sodium. La solution S₁ est une solution d'octoate de nickel dans une coupe d'hydrocarbures saturés, de point d'ébullition initial supérieur à 160 °C, contenant moins de 1 % en poids d'eau. La solution S₃ est une solution d'octoate de sodium dans une coupe d'hydrocarbures saturés, de point d'ébullition initial supérieur à 160 °C, contenant moins de 1 % en poids d'eau. Après mélange on obtient 16,5 kg d'une solution S₅ contenant 0,954 kg (16,3 moles) de nickel et 0,164 kg (7,13 moles) de sodium.

Dans un réacteur en acier inoxydable, équipé d'un système d'agitation efficace et d'un système de refroidissement performant, on introduit 6,21 kg (54,5 moles) de triéthylaluminium, puis sous pression atmosphérique et en contrôlant la température et le débit de gaz effluent la solution S₅.

La vitesse d'addition de la solution S₅ est régulée de manière à ce que la température du milieu réactionnel reste égale à la température d'ébullition du cyclohexane dans les conditions de pression choisies. Le rapport molaire Al/Ni est de 3,4. Le rapport molaire Ni/Na est de 2,3. La durée de l'addition est de deux heures. L'addition terminée, le mélange résultant est porté à 180 °C pendant 2 heures sous une pression contrôlée de 0,8 Mpa de manière à procéder à l'activation du catalyseur.

Après refroidissement et retour à la pression atmosphérique, on recueille 17 kg d'une suspension colloïdale de catalyseur dont la teneur en nickel est de 5,6 % en poids, la teneur en aluminium est de 8,7 % en poids et la teneur en sodium de 0,96 % en poids.

Une partie de cette suspension catalytique est utilisée pour hydrogéner du benzène en continu dans une petite unité pilote telle que schématisée sur la figure ci-après.

La suspension catalytique, 100 g (soit 5,6 g ou 0,095 mole de nickel et 0,96 gramme ou 0,042 mole de sodium) est introduite dans le réacteur R1 dans lequel on a préalablement placé du cyclohexane jusqu'à un niveau prédéterminé (80 % en volume du réacteur). Le contenu du réacteur est alors préchauffé sous agitation (par circulation de liquide à l'aide de la pompe P) vers 180 °C sous une pression d'hydrogène de3 Mpa.

On commence alors l'introduction par la ligne 1 de benzène (dont la teneur en soufre est de 0,2 ppm poids et la teneur en eau de 50 ppm poids) avec un débit de 108 grammes par heure (g/h) soit un débit molaire de 1,38 mole par heure. On opère la réduction dans les mêmes conditions que dans l'exemple 1. Au bout de 2 mois de fonctionnement le cyclohexane sortant du réacteur R1 par la ligne 3 contient moins de 100 ppm de benzène. La teneur en benzène du cyclohexane récupéré par la ligne 10 reste en permanence en dessous de 10 ppm pendant toute la durée du test. Le cyclohexane récupéré par la ligne 10 au bout des 2 mois de test a un point de cristallisation de 6,50 °C. Le cyclohexane récupéré contient, 280 ppm d'hydrocarbures saturés en C6 et C7 et ne contient pas de quantité détectable de méthylcyclopentane.

## Revendications

1. Procédé de production du cyclohexane, par hydrogénation du benzène, comprenant au moins les deux étapes suivantes :
a) on introduit progressivement la charge de benzène à hydrogéner et un gaz riche en hydrogène dans une zone réactionnelle contenant une phase liquide riche en cyclohexane et un catalyseur à base de nickel en suspension dans cette phase, ladite zone réactionnelle étant maintenue à une température de 100 °C à 250 °C sous une pression absolue de 0,5 MPa à 10 MPa et on récupère une phase gazeuse contenant du cyclohexane, de l'hydrogène et du benzène,
b) on introduit la phase gazeuse obtenue à l'étape a) dans un réacteur contenant au moins un lit fixe d'un catalyseur solide d'hydrogénation à base de nickel, ledit réacteur étant maintenu à une température de 100 °C à 300 °C, sous une pression absolue de 0,5 MPa à 10 MPa, la vitesse volumique horaire étant comprise entre 1 h⁻¹ et 10 h⁻¹ et on récupère après détente et refroidissement le cyclohexane sensiblement pur formé,
ledit procédé étant caractérisé en ce que le catalyseur à base de nickel employé à l'étape a) est un produit qui résulte de la réduction par au moins un trialkylaluminium de formule AlR¹R²R³ dans laquelle R¹, R² et R³ représentent chacun indépendamment les uns des autres un groupe alkyle linéaire ou ramifié ayant de 1 à 12 atomes de carbone d'une solution dans un hydrocarbure ou un mélange d'hydrocarbures, d'au moins un carboxylate de nickel de formule R⁴COONi dans laquelle R⁴ est un reste hydrocarboné ayant de 1 à 24 atomes de carbone et le plus souvent de 6 à 12 atomes de carbone, et d'au moins un carboxylate de sodium en solution de formule R⁵COONa laquelle R⁵ est un reste hydrocarboné ayant de 1 à 24 atomes de carbone, le rapport molaire Ni : Na étant compris entre 2 : 1 et 1000 : 1.

2. Procédé selon la revendication 1 dans lequel le trialkylaluminium utilisé est un composé de formule AlR¹R²R³ dans laquelle R¹, R² et R³ représentent chacun indépendamment les uns des autres un groupe alkyle linéaire ou ramifié ayant de 1 à 4 atomes de carbone, le carboxylate de nickel est un composé de formule R⁴COONi dans laquelle R⁴ est un reste hydrocarboné ayant de 6 à 12 atomes de carbone, et le carboxylate de sodium un composé de formule R⁵COONa laquelle R⁵ est un reste hydrocarboné ayant de 6 à 12 atomes de carbone.

3. Procédé selon la revendication 1 ou 2 dans lequel le trialkylaluminium utilisé est choisi dans le groupe formé par le triéthylaluminium et le triisobutylaluminium.

4. Procédé selon l'une des revendications 1 à 3 dans lequel les carboxylates de nickel et de sodium sont des carboxylates dérivant d'acides carboxyliques aliphatiques.

5. Procédé selon la revendication 4 dans lequel les carboxylates de nickel et de sodium dérivent du même acide carboxylique.

6. Procédé selon l'une des revendications 1 à 5 dans lequel les carboxylates de nickel et de sodium sont utilisés en solution dans un hydrocarbure ou un mélange d'hydrocarbures de point d'ébullition initial supérieur à 100 °C.

7. Procédé selon la revendication 6 dans lequel les carboxylates de nickel et de sodium sont utilisés en solution dans un mélange formé d'une coupe d'hydrocarbures saturés et de cyclohexane.

8. Procédé selon l'une des revendications 1 à 7 dans lequel la quantité de trialkylaluminium employée est telle que le rapport molaire aluminium sur la somme nickel plus sodium est de 1,1 : 1 à 10 : 1 et dans lequel après la première étape de réduction du sel de nickel par le composé d'aluminium la suspension est chauffée à une température supérieure à environ 100 °C sous une pression supérieure à la pression atmosphérique pendant une durée suffisante pour augmenter l'activité du catalyseur.

9. Procédé selon l'une des revendications 1 à 8 dans lequel le rapport molaire Ni :Na est compris entre 2 : 1 et 500 : 1.

10. Catalyseur à base de nickel caractérisé en ce qu'il est formé par une suspension, dans un hydrocarbure ou un mélange d'hydrocarbures, résultant de la réduction par au moins un trialkylaluminium de formule AlR¹R²R³, dans laquelle R¹, R² et R³ représentent chacun indépendamment les uns des autres un groupe alkyle linéaire ou ramifié ayant de 1 à 12 atomes de carbone, d'une solution dans un hydrocarbure ou un mélange d'hydrocarbures d'au moins un carboxylate de nickel de formule R⁴COONi, dans laquelle R⁴ est un reste hydrocarboné ayant de 1 à 24 atomes de carbone et le plus souvent de 6 à 12 atomes de carbone, et d'au moins un carboxylate de sodium en solution de formule R⁵COONa, dans laquelle R⁵ est un reste hydrocarboné ayant de 1 à 24 atomes de carbone, le rapport molaire Ni : Na étant compris entre 2 : 1 et 1000 : 1.

11. Catalyseur selon la revendication 10 dans lequel le trialkylaluminium utilisé est un composé de formule AlR¹R²R³ dans laquelle R¹, R² et R³ représentent chacun indépendamment les uns des autres un groupe alkyle linéaire ou ramifié ayant de 1 à 4 atomes de carbone, le carboxylate de nickel est un composé de formule R⁴COONi dans laquelle R⁴ est un reste hydrocarboné ayant de 6 à 12 atomes de carbone, et le carboxylate de sodium un composé de formule R⁵COONa laquelle R⁵ est un reste hydrocarboné ayant de 6 à 12 atomes de carbone.

12. Catalyseur selon la revendication 10 ou 11 dans lequel le trialkylaluminium utilisé est choisi dans le groupe formé par le triéthylaluminium et le triisobutylaluminium.

13. Catalyseur selon l'une des revendications 10 à 12 dans lequel les carboxylates de nickel et de sodium sont des carboxylates dérivant d'acides carboxyliques aliphatiques.

14. Catalyseur selon la revendication 13 dans lequel les carboxylates de nickel et de sodium dérivent du même acide carboxylique.

15. Catalyseur selon l'une des revendications 10 à 14 dans lequel les carboxylates de nickel et de sodium sont utilisés en solution dans un hydrocarbure ou un mélange d'hydrocarbures de point d'ébullition initial supérieur à 100 °C.

16. Catalyseur selon la revendication 15 dans lequel les carboxylates de nickel et de sodium sont utilisés en solution dans un mélange formé d'une coupe d'hydrocarbures saturés et de cyclohexane.

17. Catalyseur selon l'une des revendications 10 à 16 dans lequel a quantité de trialkylaluminium employée est telle que le rapport molaire aluminium sur la somme nickel plus sodium est de 1,1 : 1 à 10 : 1 et dans lequel après la première étape de réduction du sel de nickel par le composé d'aluminium, la suspension est chauffée à une température supérieure à environ 100 °C sous une pression supérieure à la pression atmosphérique pendant une durée suffisante pour augmenter l'activité du catalyseur.

18. Catalyseur selon l'une des revendications 10 à 17 dans lequel le rapport Ni : Na est compris entre 2 : 1 et 500 : 1.

## Claims

1. A process for the production of cyclohexane by hydrogenation of benzene, comprising at least two steps, as follows:
a) gradually introducing the feed of benzene to be hydrogenated and a hydrogen-rich gas into a reaction zone containing a cyclohexane-rich liquid phase and a nickel-based catalyst in suspension in said phase, said reaction zone being maintained at a temperature of 100°C to 250°C, at an absolute pressure of 0.5 MPa to 10 MPa, and recovering a gaseous phase containing cyclohexane, hydrogen and benzene;
b) introducing the gaseous phase obtained from step a) into a reactor containing at least one fixed bed of a solid nickel-based hydrogenation catalyst, said reactor being maintained at a temperature of 100°C to 300°C, at an absolute pressure of 0.5 MPa to 10 MPa, the hourly space velocity being in the range 1 h⁻¹ to 10 h⁻¹, and recovering substantially pure cyclohexane after depressurizing and cooling,
said process being characterised in that the nickel-based catalyst used in step a) is produced using at least one trialkylaluminium with formula AlR¹R²R³ where R¹, R² and R³ each independently represent a linear or branched alkyl group containing 1 to 12 carbon atoms, to reduce a solution, in a hydrocarbon or a mixture of hydrocarbons, of at least one nickel carboxylate with formula R⁴COONi, where R⁴ is a hydrocarbon residue containing 1 to 24 carbon atoms, most preferably 6 to 12 carbon atoms, and at least one sodium carboxylate with formula R⁵COONa where R⁵ is a hydrocarbon residue containing 1 to 24 carbon atoms, the Ni:Na molar ratio being between 2:1 and 1000:1.

2. A process according to claim 1, in which the trialkylaluminium is a compound with formula AlR¹R²R³ where R¹, R² and R³ each independently represent a linear or branched alkyl group containing 1 to 4 carbon atoms, the nickel carboxylate is a compound with formula R⁴COONi where R⁴ is a hydrocarbon residue containing 6 to 12 carbon atoms, and the sodium carboxylate is a compound with formula R⁵COONa where R⁵ is a hydrocarbon residue containing 6 to 12 carbon atoms.

3. A process according to claim 1 or claim 2, in which the trialkylaluminium used is selected from the group formed by triethylaluminium and triisobutylaluminium.

4. A process according to any one of claims 1 to 3, in which the nickel and sodium carboxylates are derived from aliphatic carboxylic acids.

5. A process according to claim 4, in which the nickel and sodium carboxylates derive from the same carboxylic acid.

6. A process according to any one of claims 1 to 5, in which the nickel and sodium carboxylates are used in solution in a hydrocarbon or a mixture of hydrocarbons with an initial boiling point of more than 100°C.

7. A process according to claim 6, in which the nickel and sodium carboxylates are used in solution in a mixture formed by a saturated hydrocarbon cut and cyclohexane.

8. A process according to any one of claims 1 to 7, in which the quantity of trialkylaluminium used is such that the molar ratio of aluminium to the sum of the nickel and sodium is 1.1:1 to 10:1 and in which after the first step for reducing the nickel salt by the aluminium compound, the suspension is heated to a temperature above about 100°C at a pressure above atmospheric pressure for a period sufficient to increase the activity of the catalyst.

9. A process according to any one of claims 1 to 8, in which the Ni:Na molar ratio is in the range 2:1 to 500:1.

10. A nickel-based catalyst, characterised in that it is formed by a suspension, in a hydrocarbon or a mixture of hydrocarbons, produced by using at least one trialkylaluminium with formula AlR¹R²R³ where R¹, R² and R³ each independently represent a linear or branched alkyl group containing 1 to 12 carbon atoms, to reduce a solution, in a hydrocarbon or a mixture of hydrocarbons, of at least one nickel carboxylate with formula R⁴COONi, where R⁴ is a hydrocarbon residue containing 1 to 24 carbon atoms, most preferably 6 to 12 carbon atoms, and at least one sodium carboxylate with formula R⁵COONa where R⁵ is a hydrocarbon residue containing 1 to 24 carbon atoms, the Ni:Na molar ratio being in the range 2:1 to 1000:1.

11. A catalyst according to claim 1, in which the trialklyalumimum used is a compound with formula AlR¹R²R³ where R¹, R² and R³ each independently represents a linear or branched alkyl group containing 1 to 4 carbon atoms, the nickel carboxylate is a compound with formula R⁴COONi, where R⁴ is a hydrocarbon residue containing 6 to 12 carbon atoms, and the sodium carboxylate is a compound with formula R⁵COONa where R⁵ is a hydrocarbon residue containing 6 to 12 carbon atoms.

12. A catalyst according to claim 10 or claim 11, in which the trialkylaluminium used is selected from the group formed by triethylaluminium and triisobutylaluminium.

13. A catalyst according to any one of claims 10 to 12, in which the nickel and sodium carboxylates are derived from aliphatic carboxylic acids.

14. A catalyst according to claim 13, in which the nickel and sodium carboxylates derive from the same carboxylic acid.

15. A catalyst according to any one of claims 10 to 14, in which the nickel and sodium carboxylates are used in solution in a hydrocarbon or a mixture of hydrocarbons with an initial boiling point of more than 100°C.

16. A catalyst according to claim 15, in which the nickel and sodium carboxylates are used in solution in a mixture formed by a saturated hydrocarbon cut and cyclohexane.

17. A catalyst according to any one of claims 10 to 16, in which the quantity of trialkylaluminium used is such that the molar ratio of aluminium to the sum of the nickel and sodium is 1.1:1 to 10:1 and in which after the first reduction step for the nickel salt by the aluminium compound, the suspension is heated to a temperature above about 100°C at a pressure above atmospheric pressure for a period sufficient to increase the activity of the catalyst.

18. A catalyst according to any one of claims 10 to 17, in which the Ni:Na molar ratio is in the range 2:1 to 500:1.

## Patentansprüche

1. Verfahren zur Erzeugung von Cyclohexan durch Hydrierung von Benzen, das wenigstens die zwei folgenden Schritte umfaßt:
a) progressives Einführen der zu hydrierenden Benzencharge und eines wasserstoffreichen Gases in eine Reaktionszone, die eine an Cyclohexan reiche flüssige Phase und einen Katalysator auf Nickelgrundlage in Suspension in dieser Phase enthält, wobei die Reaktionszone auf einer Temperatur von 100°C bis 250°C unter einen Absolutdruck von 0,5 MPa bis 10 MPa gehalten wird, und Auffangen einer Cyclohexan, Wasserstoff und Benzen enthaltenden gasförmigen Phase,
b) Einführung der in Schritt a) erhaltenen gasförmigen Phase in einen Reaktor, der wenigstens ein Festbett eines festen Hydrierkatalysators auf Nickelgrundlage enthält, wobei der Reaktor auf eine Temperatur von 100°C bis 300°C unter einen Absolutdruck von 0,5 MPa bis 10 MPa gehalten wird, wobei die Volumengeschwindigkeit zwischen 1h⁻¹ und 10h⁻¹ gehalten wird, und Auffangen des gebildeten, im wesentlichen reinen Cyclohexans nach Entspannung und Abkühlung,
dadurch gekennzeichnet, daß der in Schritt a) verwendete Katalysator auf Nickelgrundlage
a) ein Erzeugnis ist, welches resultiert aus der Reduktion durch wenigstens ein Trialkylaluminium der Formel AlR¹R²R³, wobei R¹, R² und R³ jeweils unabhängig voneinander eine lineare oder verzweigte Alkylgruppe mit 1 bis 12 Kohlenstoffatomen darstellen, einer Lösung in einem Kohlenwasserstoff oder einem Kohlenwasserstoffgemisch von wenigstens einem Nickelcarboxylat der Formel R⁴COONi, wobei R⁴ ein Kohlenwasserstoffrest mit 1 bis 24 Kohlenstoffatomen und meist mit 6 bis 12 Kohlenstoffatomen ist, und wenigstens einem Natriumcarboxylat in Lösung mit der Formel R⁵COONa, wobei R⁵ ein Kohlenwasserstoffrest mit 1 bis 24 Kohlenstoffatomen ist und das Molverhältnis Ni:Na zwischen 2:1 und 1000:1 enthalten ist.

2. Verfahren nach Anspruch 1, bei dem das verwendete Trialkylaluminium eine Verbindung der Formel AlR¹R²R³ ist, wobei R¹, R² und R³ jeweils unabhängig voneinander eine lineare oder verzweigte Alkylgruppe mit 1 bis 4 Kohlenstoffatomen darstellen, das Nickelcarboxylat eine Verbindung der Formel R⁴COONi ist, wobei R⁴ ein Kohlenwasserstoffrest mit 6 bis 12 Kohlenstoffatomen ist, und das Natriumcarboxylat eine Verbindung der Formel R⁵COONa ist, wobei R⁵ ein Kohlenwasserstoffrest mit 6 bis 12 Kohlenstoffatomen ist.

3. Verfahren nach Anspruch 1 oder 2, bei dem das verwendete Trialkylaluminium in der Gruppe, gebildet durch Triethylaluminium und Triisobutylaluminium, gewählt ist.

4. Verfahren nach einem der Ansprüche 1 bis 3, bei dem die Carboxylate von Nickel und Natrium von aliphatischen Carboxylsäuren ableitende Carboxylate sind.

5. Verfahren nach Anspruch 4, bei dem die Carboxylate von Nickel und Natrium von derselben Carboxylsäure ableiten.

6. Verfahren nach einem der Ansprüche 1 bis 5, bei dem die Carboxylate von Nickel und Natrium in einem Kohlenwasserstoff oder Gemisch von Kohlenwasserstoffen mit anfänglichem Siedepunkt oberhalb 100°C verwendet werden.

7. Verfahren nach Anspruch 6, bei dem die Carboxylate von Nickel und Natrium in Lösung in einem Gemisch verwendet werden, das aus einem Schnitt von gesättigten Kohlenwasserstoffen und Cyclohexan gebildet ist.

8. Verfahren nach einem der Ansprüche 1 bis 7, bei dem die verwendete Menge an Trialkylaluminium so ist, daß das Molverhältnis Aluminium zur Summe Nickel plus Natrium 1,1:1 bis 10:1 beträgt, und bei dem nach dem ersten Schritt der Reduktion des Nickelsalzes durch die Aluminiumverbindung die Suspension auf eine Temperatur oberhalb ca. 100°C unter überatmosphärischem Druck für eine zum Erhöhen der Aktivität des Katalysators ausreichende Zeitdauer erhitzt wird.

9. Verfahren nach einem der Ansprüche 1 bis 8, bei dem das Molverhältnis Ni:Na zwischen 2:1 und 500:1 enthalten ist.

10. Katalysator auf Nickelgrundlage, dadurch gekennzeichnet, daß er durch eine Suspension in einem Kohlenwasserstoff oder einem Gemisch von Kohlenwasserstoffen gebildet ist, resultierend aus der Reduktion durch wenigstens ein Trialkylaluminium der Formel AlR¹R²R³, wobei R¹, R² und R³ jeweils unabhängig voneinander eine lineare oder verzweigte Alkylgruppe mit 1 bis 12 Kohlenstoffatomen darstellen, einer Lösung in einem Kohlenwasserstoff oder einem Gemisch von Kohlenwasserstoffen wenigstens eines Nickelcarboxylates der Formel R⁴COONi, wobei R⁴ ein Kohlenwasserstoffrest mit 1 bis 24 Kohlenstoffatomen und meist 6 bis 12 Kohlenstoffatomen ist, und wenigstens eines Natriumcarboxylates in Lösung mit der Formel R⁵COONa, wobei R⁵ ein Kohlenwasserstoff mit 1 bis 24 Kohlenstoffatomen ist, wobei das Molverhältnis Ni:Na zwischen 2:1 und 1000:1 enthalten ist.

11. Katalysator nach Anspruch 10, bei dem das verwendete Trialkylaluminium eine Verbindung der Formel AlR¹R²R³ ist, wobei R¹, R² und R³ jeweils unabhängig voneinander eine lineare oder verzweigte Alkylgruppe mit 1 bis 4 Kohlenstoffatomen darstellen, das Nickelcarboxylat eine Verbindung der Formel R⁴COONi ist, wobei R⁴ ein Kohlenwasserstoffrest mit 6 bis 12 Kohlenstoffatomen ist, und das Natriumcarboxylat eine Verbindung der Formel R⁵COONa ist, wobei R⁵ ein Kohlenwasserstoffrest mit 6 bis 12 Kohlenstoffatomen ist.

12. Katalysator nach Anspruch 10 oder 11, bei dem das verwendete Trialkylaluminium in der durch Triethylaluminium und Triisobutylaluminium gebildeten Gruppe gewählt ist.

13. Katalysator nach einein der Ansprüche 10 bis 12, bei dem die Carboxylate von Nickel und Natrium von aliphatischen Carboxylsäuren ableitende Carboxylate sind.

14. Katalysator nach Anspruch 13, bei dem die Carboxylate von Nickel und Natrium von derselben Carboxylsäure ableiten.

15. Katalysator nach einem der Ansprüche 10 bis 14, bei dem die Carboxylate von Nickel und Natrium in Lösung in einein Kohlenwasserstoft oder einein Gemisch von Kohlenwasserstoffen mit anfänglichem Siedepunkt oberhalb 100°C verwendet werden.

16. Katalysator nach Anspruch 15, bei dem die Carboxylate von Nickel und Natrium in Lösung in einein Gemisch, gebildet aus einem Schnitt von gesättigtem Kohlenwasserstoffen und Cyclohexan, verwendet werden.

17. Katalysator nach einem der Ansprüche 10 bis 16, bei dem die verwendete Menge an Trialkylaluminium derart ist, daß das Molverhältnis Aluminium zur Summe Nickel plus Natrium von 1,1:1 bis 10:1 beträgt, und bei dem nach dem ersten Schritt der Reduktion des Nickelsalzes durch die Aluminiumverbindung die Suspension auf eine Temperatur oberhalb ca. 100°C unter einem überatmosphärischen Druck für eine zum Erhöhen der Aktivität des Katalysators ausreichende Zeitdauer erhitzt ist.

18. Katalysator nach einem der Ansprüche 10 bis 17, bei dem das Verhältnis Ni:Na zwischen 2:1 und 500:1 enthalten ist.
